# EUROPEAN PATENT APPLICATION

(11) **EP 3 118 856 A1**
(43) Date of publication of application: **18.01.2017**
(21) Application number: 15762281.2
(22) Date of filing: 10.03.2015
(51) Int. Cl.: G21F 9/18, G21F 9/28, G21F 9/32, B01D 50/00, B01D 53/34, C12N 1/00

(54) **RADIOACTIVE DECONTAMINATION DEVICE AND DECONTAMINATION METHOD**

(30) Priority: 14.03.2014 JP 2014051632
(71) Applicant: Sou, Yuichi, Sendai-shi, Miyagi 983-0002 (JP); Niibori, Yasuhiro, Tokyo 160-0012 (JP)
(72) Inventor: Sou, Yuichi, Sendai-shi, Miyagi 983-0002 (JP)
(74) Representative: D Young & Co LLP
(86) International application number: PCT/JP2015/056944
(87) International publication number: WO 2015/137311

(57) **Abstract**

Disclosed are a radioactive decontamination device and a radioactive decontamination method, each capable of efficiently and overall reducing the radioactivity level of a radioactive contaminant.

The present invention provides a radioactive decontamination device for reducing the radioactivity level of a carbonized radioactive contaminant (B) having a radioactive material, which includes a stirring container (41) for stirring the carbonized radioactive contaminant (B), a discharge feeder (42) for discharging the stirred carbonized radioactive contaminant (B) which is disposed below the stirring container (41), wherein the stirring container (41) is provided with a nozzle (45) for spraying an aqueous solution (44) containing photosynthetic bacteria over the carbonized radioactive contaminant (B) to be fed into the stirring container (41).

## Description

### TECHNICAL FIELD

The present invention relates to a radioactive decontamination device and a radioactive decontamination method, each capable of efficiently reducing the radioactivity level of a radioactive contaminant having a radioactive material.

### BACKGROUND

The occurrence of accidental explosions at nuclear power plants inevitably causes radioactive fallout, typically comprised of iodine 131, cesium 137 and strontium 90. While such radioactive materials subsequently contaminate the neighboring areas, they unfortunately spread and even travel farther as airborne contaminants. Of these, cesium 137, with a long half-life of approximately 30 years, is particularly problematic to remove from the contaminated soil. Merely washing away the contaminated soil is not enough to completely reduce the environmentally damaging radioactivity level. This problem renders decontamination processes difficult.

One recent technique, however, has succeeded in developing a radioactive decontamination method for reducing the radioactivity level of affected land and articles by spraying the same with photosynthetic bacteria (Patent Document 1).

### PRIOR ART DOCUMENTS

### PATENT DOCUMENTS

Patent Document 1: JP-A-2013-130574

### SUMMARY OF THE INVENTION

### PROBLEM TO BE SOLVED BY THE INVENTION

However, in the above prior-art radioactive decontamination method, the process of spraying such photosynthetic bacteria fails to completely reduce the radioactivity level of contaminated fallen leaves or straw accumulated in layers or a large mass of radioactive waste.

It is an object of the present invention to provide a radioactive decontamination device capable of efficiently and overall reducing the radioactivity level of a radioactive contaminant to be decontaminated.

### MEANS FOR SOLVING THE PROBLEM

To solve the above-mentioned problems, in accordance with a first aspect of the present invention, a radioactive decontamination device for reducing the radioactivity level of a radioactive contaminant having a radioactive material comprises: a stirring container for stirring the radioactive contaminant; and a discharge feeder for discharging the stirred radioactive contaminant which is disposed below the stirring container, wherein the stirring container is provided with a nozzle for spraying an aqueous solution containing photosynthetic bacteria over the radioactive contaminant to be fed into the stirring container.

In accordance with a second aspect of the present invention, the radioactive decontamination device according to the first aspect of the invention is characterized in that the photosynthetic bacteria is at least one of purple sulfur bacteria, purple nonsulfur bacteria and green sulfur bacteria.

In accordance with a third aspect of the present invention, the radioactive decontamination device according to the first or second aspect of the invention is characterized in that the stirring container can be retained in an oxygen-starved state.

In accordance with a fourth aspect of the present invention, the radioactive decontamination device according to any one of the first to third aspects of the invention is characterized in that the radioactive contaminant is a carbonized radioactive contaminant through a carbonization process in a thermal decomposition incinerator.

In accordance with a fifth aspect of the present invention, the radioactive decontamination device according to the fourth aspect of the invention is characterized in that the thermal decomposition incinerator comprises a heating container for stirring and heating the radioactive contaminant in a sealed state.

In accordance with a sixth aspect of the present invention, the radioactive decontamination device according to the fourth or fifth aspect of the invention further comprises a deodorizing apparatus for removing the radioactive material and odor from exhaust gas generated in the thermal decomposition incinerator by introducing the exhaust gas and allowing the introduced exhaust gas to pass through a first space having sprayed water falling down and a second space having porous materials in sequence.

In accordance with a seventh aspect of the present invention, the radioactive decontamination device according to the sixth aspect of the invention further comprises a smoke removing apparatus for removing smoky microparticles from exhaust gas discharged from the deodorizing apparatus by introducing the exhaust gas and allowing the introduced exhaust gas to pass through stored water and then a smoke removing filter.

In accordance with an eighth aspect of the present invention, a radioactive decontamination method for reducing the radioactivity level of a radioactive contaminant having a radioactive material comprises the steps of: spraying the radioactive contaminant with an aqueous solution containing photosynthetic bacteria; stirring the radioactive contaminant for a predetermined period of time; filling a container with the stirred radioactive contaminant; and storing the same for a predetermined period of time to reduce the radioactivity level of the radioactive contaminant.

In accordance with a ninth aspect of the present invention, the radioactive decontamination method according to the eighth aspect of the invention is characterized in that the photosynthetic bacteria is at least one of purple sulfur bacteria, purple nonsulfur bacteria and green sulfur bacteria.

In accordance with a tenth aspect of the present invention, the radioactive decontamination method according to the eighth or ninth aspect of the invention is characterized in that the radioactive contaminant is stirred and stored in an oxygen-starved state.

In accordance with an eleventh aspect of the present invention, the radioactive decontamination method according to any one of the eighth to tenth aspects of the invention is characterized in that the radioactive contaminant is a carbonized radioactive contaminant through a carbonization process.

### EFFECT OF THE INVENTION

In accordance with the first aspect of the present invention, the radioactive decontamination device for reducing the radioactivity level of a radioactive contaminant having a radioactive material comprises a stirring container for stirring the radioactive contaminant and a nozzle for spraying an aqueous solution containing photosynthetic bacteria over the radioactive contaminant to be fed into the stirring container. This property enables photosynthetic bacteria to uniformly be dispersed within the radioactive contaminant. Accordingly, the photosynthetic bacteria allows the radioactivity level of the radioactive material within even a large amount of the radioactive contaminant to be uniformly and efficiently reduced.

The radioactivity level of the radioactive contaminant, which is stirred to uniformly disperse the photosynthetic bacteria therewithin, remains high. However, a discharge feeder which is disposed below the stirring container allows the radioactive contaminant to be handled safely without direct contact and filled in a storage container. In a test measurement, the radioactivity level of such a radioactive contaminant stored in a container was 3500 Bq/Kg upon the start of storage, but the subsequent action of the photosynthetic bacteria reduced the value to 81 Bq/Kg within two months. Accordingly, this test shows a significant effect of reducing the radioactivity level to under 100 Bq/Kg, a standard radioactive level of general food products within two months.

In accordance with the second aspect of the present invention, the radioactive decontamination device is characterized in that the photosynthetic bacteria is at least one of purple sulfur bacteria, purple nonsulfur bacteria and green sulfur bacteria as photosynthetic bacteria which is readily available at low cost and useful in reducing costs required for decontaminating a radioactive material. The above types of bacteria may be used singularly or in combination. Of these, the purple nonsulfur bacteria is particularly capable of reducing the radioactivity level.

In accordance with the third aspect of the present invention, the radioactive decontamination device is characterized in that the stirring container can be retained in an oxygen-starved state. This property enables an anaerobe such as purple sulfur bacteria, purple nonsulfur bacteria and green sulfur bacteria to grow in a favorable environment, thereby advantageously improving the effect of decontaminating a radioactive material. The oxygen-starved state can be achieved by preparing sucking means for sucking air from a stirring container (e.g., vacuum pump) and atmospheric gas supply means for supplying gas containing no oxygen (atmospheric gas) such as nitrogen, carbon dioxide and vapor to the stirring container.

In accordance with the fourth aspect of the present invention, the radioactive decontamination device is characterized in that the radioactive contaminant is a carbonized radioactive contaminant through a carbonization process in a thermal decomposition incinerator. This property enables the radioactive contaminant to be decontaminated using photosynthetic bacteria, with increased density and intensity of the radioactivity of the radioactive contaminant. Accordingly, the radioactivity level of the radioactive contaminant can extremely efficiently be reduced.

In accordance with the fifth aspect of the present invention, the radioactive decontamination device is characterized in that the thermal decomposition incinerator includes a heating container for stirring and heating the radioactive contaminant in a sealed state. This property can prevent the release of exhaust gas generated in the process of carbonization by a thermal decomposition into the atmosphere, as well as the radioactive material.

In accordance with the sixth aspect of the present invention, the radioactive decontamination device further comprises a deodorizing apparatus for removing the radioactive material and odor from exhaust gas generated in the thermal decomposition incinerator by introducing the exhaust gas and allowing the introduced exhaust gas to pass through a first space having sprayed water falling down and a second space having porous materials. Accordingly, discharge of the radioactive material and odor into the atmosphere can be prevented, as well as the exhaust gas generated in the thermal decomposition incinerator. The radioactivity level of the radioactive material contained in sprayed water and the radioactive material on porous materials can readily be reduced using photosynthetic bacteria.

In accordance with the seventh aspect of the present invention, the radioactive decontamination device according to the first aspect of the invention further comprises a deodorizing apparatus for removing smoky microparticles from exhaust gas discharged from the deodorizing apparatus by introducing the exhaust gas and allowing the introduced exhaust gas to pass through stored water and then a smoke removing filter. Accordingly, the exhaust gas can be discharged into the atmosphere by removing fine dust such as smoke contained in the exhaust gas generated in the thermal decomposition incinerator.

In accordance with the eighth aspect of the present invention, the radioactive decontamination method for reducing the radioactivity level of a radioactive contaminant having a radioactive material comprises the steps of: spraying the radioactive contaminant with an aqueous solution containing photosynthetic bacteria; stirring the radioactive contaminant for a predetermined period of time; filling a container with the stirred radioactive contaminant; and storing the same for a predetermined period of time. This method can considerably readily decontaminate a radioactive contaminant.

In accordance with the ninth aspect of the present invention, the radioactive decontamination method is characterized in that the photosynthetic bacteria is at least one of purple sulfur bacteria, purple nonsulfur bacteria and green sulfur bacteria. This property is useful in reducing costs for decontaminating a radioactive material.

In accordance with the tenth aspect of the present invention, the radioactive decontamination method is characterized in that the radioactive contaminant is stirred and stored in an oxygen-starved state to favorably maintain the environment for growing photosynthetic bacteria of at least one of purple sulfur bacteria, purple nonsulfur bacteria and green sulfur bacteria as an anaerobe, thereby improving the effect of reducing the radioactivity level.

In accordance with the eleventh aspect of the present invention, the radioactive decontamination method is characterized in that the radioactive contaminant is a carbonized radioactive contaminant through a carbonization process. This property enables the radioactive contaminant, with increased density and intensity of the radioactivity, to be decontaminated using photosynthetic bacteria. Accordingly, the intensity of the radioactivity level of a radioactive contaminant can extremely efficiently be reduced.

### BRIEF DESCRIPTION OF THE DRAWINGS

FIG. 1 is a block diagram illustrating a radioactive decontamination device of one embodiment according to the present invention;
FIG. 2 is an elevation view illustrating a thermal decomposition incinerator in the radioactive decontamination device, with main parts indicated in dashed line;
FIG. 3 is an elevation view illustrating a deodorizing apparatus in the radioactive decontamination device, with main parts indicated in dashed line;
FIG. 4 is an elevation view illustrating a smoke removing apparatus in the radioactive decontamination device, with main parts indicated in dashed line;
FIG. 5 is an elevation view illustrating a stirring container, a discharge feeder and a nozzle in the radioactive decontamination device, with main parts indicated in dashed line; and
FIG. 6 is a cross-sectional view taken from line VI - VI of FIG. 5 illustrating a stirring container, a discharge feeder and a nozzle in the radioactive decontamination device.

### DESCRIPTION OF THE PREFERRED EMBODIMENTS

An embodiment of the radioactive decontamination device according to the present invention will be described with reference to the drawings.

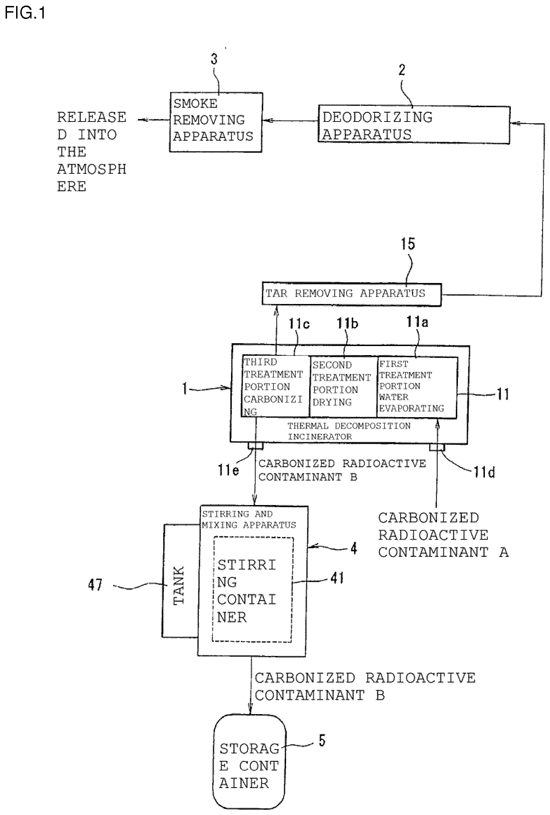

The radioactive decontamination device according to this embodiment, as shown in FIG. 1, : a thermal decomposition incinerator 1 for carbonizing a radioactive contaminant "A"; a tar removing apparatus 15 for removing a tar component from exhaust gas generated in the thermal decomposition incinerator 1; a deodorizing apparatus 2 for removing a radioactive material and odor from exhaust gas discharged from the tar removing apparatus 15 by introducing the exhaust gas; a smoke removing apparatus 3 for removing smoky microparticles from exhaust gas discharged from the deodorizing apparatus 2 by introducing the exhaust gas; and a stirring and mixing apparatus 4 for stirring and mixing with photosynthetic bacteria a carbonized radioactive contaminant "B" through a carbonization process in the thermal decomposition incinerator 1 to be fed.

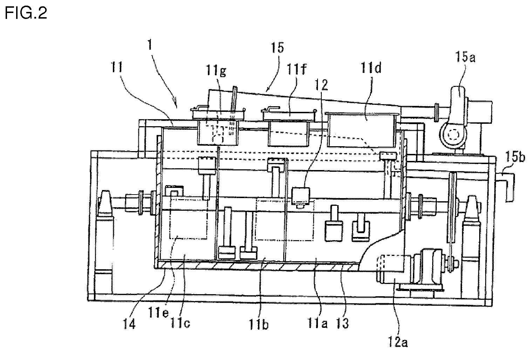

The thermal decomposition incinerator 1, as shown in FIG. 2, comprises a heating container 11 for stirring and heating the radioactive contaminant "A" in a sealed state. Specifically, the heating container 11 is divided into a first treatment portion 11a, a second treatment portion 11b and a third treatment portion 11c. The first treatment portion 11a is provided with a charging port 11d for the radioactive contaminant "A", while the third treatment portion 11c is provided with a discharge port 11e. The heating container 11 further comprises stirring means 12 for stirring the radioactive contaminant "A" to be fed and transporting the same from the first treatment portion 11a to the third treatment portion 11c in sequence. The stirring means 12 is configured to be rotationally driven by a motor 12a. Preferably, the radioactive contaminant "A" may be reduced in volume by carbonization, such as fallen leaves, straw, radioactive contamination protective clothing and sewage sludge, each contaminated with radiation.

On the outer peripheral surface of the heating container 11 is attached a heater 13, which enables the first treatment portion 11a to evaporate water in the radioactive contaminant "A, " the second treatment portion 11b to primarily dry and heat the radioactive contaminant "A," and the third treatment portion 11c to carbonize the radioactive contaminant "A" by a thermal decomposition. The heater 13 is externally coated with an insulating plate 14.

A tar removing apparatus 15 is disposed so as to be adjacent to the heating container 11. The tar removing apparatus 15 is configured to collect tar in the form of liquid by introducing exhaust gas generated in the heating container 11 to the interior by a blower 15a and cooling and condensing the exhaust gas by a capture plate disposed within the heating container 11 (not shown). Collected tar can be discharged from a discharge pipe 15b to the exterior.

In FIG. 1, numerals 11f and 11g refer to inspection openings.

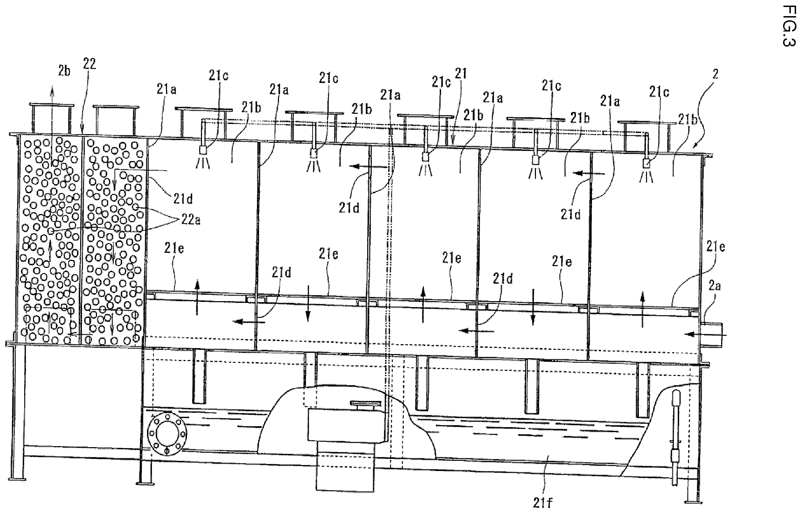

The deodorizing apparatus 2, as shown in FIG. 3, is configured to remove the radioactive material and odor from exhaust gas after removing a tar component in the tar removing apparatus 15 by introducing the exhaust gas and allowing the exhaust gas to pass through a first space 21 having sprayed water falling down and a second space 22 filled with a plurality of porous materials 22a in sequence.

The first space 21 having sprayed water falling down is divided into a plurality of chambers 21b by a plurality of partition plates 21a vertically installed. Each of the chambers 21b is provided on a ceiling portion thereof with a shower head 21c for spraying water.

Each of the partition plates 21a is provided with an opening 21d near the upper or lower end thereof. In the deodorizing apparatus 2, two adjacent partition plates 21a, one having an opening 21d near the upper end thereof and the other having an opening 21d near the lower end thereof, are arranged to define the chamber 21b. Each of the chambers 21b is configured to consist of upper and lower portions. These portions are vertically divided by a porous plate 21e placed slightly above the opening 21d near the lower end of the partition plate 21a. A water storage tank 21f, which is disposed below the porous plate 21e, is configured to receive water sprayed from the shower head 21c after passing through the porous plate 21e. Exhaust gas introduced through the tar removing apparatus 15 vertically moves to the chamber 21b in sprayed water through the porous plate 21e, and further moves to an adjacent chamber 21b through an opening 21d near the upper or lower end of the partition plate 21a. Subsequently, the radioactive material in the exhaust gas, which is attached to water, is transported to the water storage tank 21f.

The radioactive material contained in the exhaust gas transported from the first space 21 to the second space 22 is adsorbed onto the porous materials 22a. Each of the porous materials 22a is formed of a mineral for adsorption, particularly mudstone capable of high adsorption of a radioactive material, toxic gases, etc. In FIG. 3, numerals 2a and 2b represent an inlet of the exhaust gas from the tar removing apparatus 15 and a discharge port of exhaust gas from the second space 22, respectively.

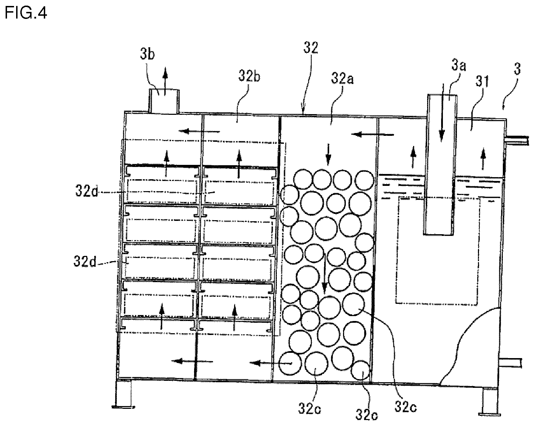

The smoke removing apparatus 3, as shown in FIG. 4, is configured to remove smoky microparticles and a radioactive material from exhaust gas discharged from a discharge port 2b of the deodorizing apparatus 2 by introducing the exhaust gas, and allowing the introduced exhaust gas to pass through water stored in a water tank 31, and then first and second smoke removing filter portions (smoke removing filters) 32a, 32b of a filter chamber 32 in sequence. The first smoke removing filter portion 32a for first feeding exhaust gas is configured to capture and adsorb the microparticles, the radioactive material, toxic gases, etc. by a number of porous materials 32c provided therewithin. Meanwhile, the second smoke removing filter portion 32b includes a plurality of filter members 32d formed of fibers and activated charcoal therewithin to capture and adsorb the microparticles, the radioactive material, toxic gases, etc. by the filter members 32d.

Each of the porous materials 32c is formed of a mineral for adsorption, particularly mudstone capable of high adsorption of a radioactive material, toxic gases, etc. In FIG. 4, numerals 3a and 3b represent an inlet of the exhaust gas from the deodorizing apparatus 2 and a discharge port of exhaust gas from the second smoke removing filter portion 32b, respectively.

Accordingly, the exhaust gas generated in the thermal decomposition incinerator 1 is treated as completely harmless and released into the atmosphere through the tar removing apparatus 15, the deodorizing apparatus 2 and the smoke removing apparatus 3.

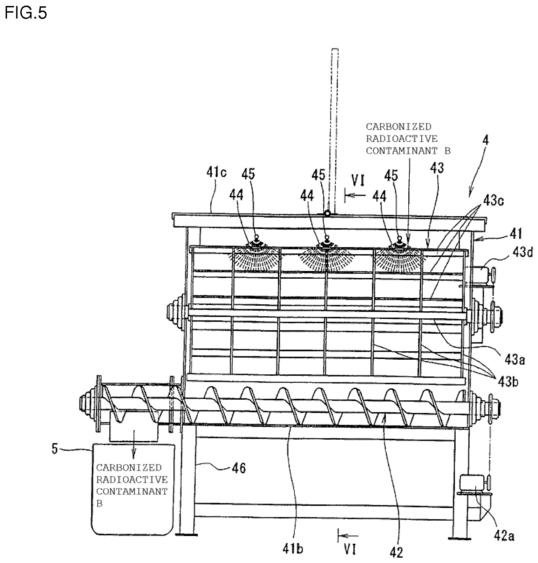

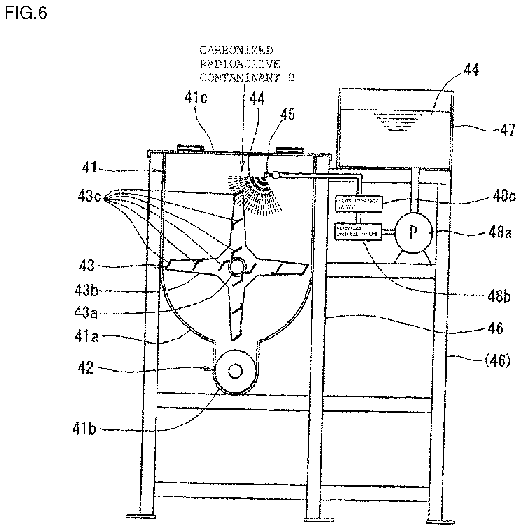

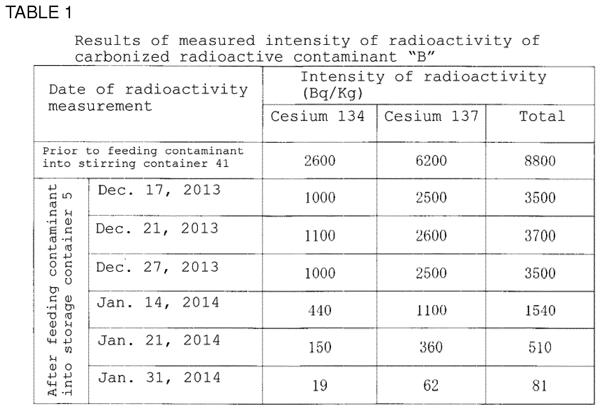

The stirring and mixing apparatus 4, as shown in FIGS. 5 and 6, comprises a stirring container 41 for stirring a carbonized radioactive contaminant "B" to be fed, and a discharge feeder 42 for discharging the stirred carbonized radioactive contaminant "B" to the exterior which is disposed below the stirring container 41.

The stirring container 41 comprises a bottom surface portion 41a which is formed of a semi-circular cylinder and a bulge portion 41b formed by protruding the central portion of the bottom surface portion 41a further downward. The bottom portion of the bulge portion 41b is also formed of a semi-circular cylinder. The upper portion of the stirring container 41 may be a quadrangular opening, and is provided with a lid 41c. The lid 41c is capable of opening and closing the opening half and half by a hinge provided at the central portion. While the opening is closed, the opening is closed to seal the stirring container 41.

The stirring container 41 is provided with stirring means 43 for stirring a carbonized radioactive contaminant "B" to be fed. The stirring means 43 comprises a rotary shaft 43a, a spoke portion 43b axially secured to the rotary shaft 43a at predetermined intervals and a plurality of stirring plates 43c disposed so as to overlie each of the adjacent spoke portions 43b. Each of the spoke portions 43b is formed of a cruciform plate-like member. The stirring means 43 thus configured is rotationally driven by a motor 43d.

Further, the stirring container 41 is provided with a nozzle 45 for spraying an aqueous solution 44 containing photosynthetic bacteria over the carbonized radioactive contaminant "B" to be fed into the stirring container 41. A plurality of nozzles 45 are axially spaced at predetermined intervals relative to the stirring means 43 (three nozzles used in this embodiment). The photosynthetic bacteria may be at least one of purple sulfur bacteria, purple nonsulfur bacteria and green sulfur bacteria. The purple nonsulfur bacteria is preferably used in this embodiment due to its high capability to reduce the radioactivity level.

A frame 46 for stably retaining the stirring container 41 is provided with a tank 47 for storing the aqueous solution 44 containing the photosynthetic bacteria, and a pump 48a for feeding the aqueous solution 44 stored in the tank 47 to the nozzle 45, a pressure control valve 48b and a flow control valve 48c. The pressure control valve 48b is capable of adjusting the pressure of the aqueous solution 44 to be fed into the nozzle 45. The pressure control valve 48b may be manually adjusted or automatically controlled by an electromagnetic system. The flow control valve 48c is capable of adjusting the flow of the aqueous solution 44 to be fed into the nozzle 45. The flow control valve 48c may be manually adjusted or electromagnetically adjusted.

The discharge feeder 42 is formed of a screw shaft, and rotationally driven by a motor 42a. Accordingly, in the stirring container 41, the carbonized radioactive contaminant "B, " which is stirred and mixed with the photosynthetic bacteria, can be discharged to the exterior of the stirring container 41.

The carbonized radioactive contaminant "B" discharged by the discharge feeder 42 is continuously fed into a storage container 5. The storage container 5 may be, e.g., a flexible container bag or a ton bag. The storage container 5 is configured to store the carbonized radioactive contaminant "B" until its radioactivity level is reduced to a predetermined intensity (e.g., 100 Bq/Kg) by the photosynthetic bacteria.

Then, one embodiment of the radioactive decontamination method according to the present invention will be described. This radioactive decontamination method comprises the steps of: spraying an aqueous solution 44 containing photosynthetic bacteria over a carbonized radioactive contaminant "B"; stirring the carbonized radioactive contaminant "B" for a predetermined period of time (approximately 10 to 40 minutes) ; filling a storage container 5 with the stirred carbonized radioactive contaminant "B"; and storing the same for a predetermined period of time (approximately 1 and half months to 2 months) to reduce the radioactivity level of the carbonized radioactive contaminant "B."

Subsequently, the test examples of the present invention will be described.

### 1. Test conditions

### (1) Date of test

(i) Date of feeding radioactive contaminant "A" into thermal decomposition incinerator 1
   Dec. 16, 2013
(ii) Date of stirring and mixing with photosynthetic bacteria carbonized radioactive contaminant "B" by stirring and mixing apparatus 4
   Dec. 17, 2013

(1) Type of radioactive contaminant "A"
   Fallen leaves collected in Kawamata Town, Fukushima Prefecture
(2) Amount of radioactive contaminant "A" to be fed into thermal decomposition incinerator 1
   11Kg
(3) Amount of carbonized radioactive contaminant "B" to be fed into stirring container 41
   (Equivalent to the amount of carbonized radioactive contaminant "B" discharged from thermal decomposition incinerator 1)
   2.2Kg
(4) Total amount of aqueous solution containing photosynthetic bacteria to be fed
   1.5 liter
(5) Stirring time in stirring and mixing apparatus 4 30 minutes
(6) Conditions of storing stirred and mixed carbonized radioactive contaminant "B"

A carbonized radioactive contaminant "B" was filled in a flexible container bag as a storage container 5 and then stored in a weather-proof environment.

### 2. Test method

The intensity of the radioactivity of the above carbonized radioactive contaminant "B" was measured prior to feeding the same into the stirring container 41 and after feeding the same into the storage container 5. After feeding the contaminant "B" into the storage container 5, the intensity was measured several times.

### 3. Test results

### [Table 1]

The above test results show that the radioactivity level of general food products specified by the Ministry of Health, Labour and Welfare was under a standard radioactive level of cesium (100 Bq/Kg), approximately one and half months even from the date of feeding a radioactive contaminant "A" into a thermal decomposition incinerator 1 (Dec. 16, 2013).

The above-configured radioactive decontamination device comprises a stirring container 41 for stirring a carbonized radioactive contaminant "B" as a radioactive contaminant and a nozzle 45 for spraying an aqueous solution 44 containing photosynthetic bacteria over the carbonized radioactive contaminant "B" to be fed into the stirring container 41. This property enables the photosynthetic bacteria to uniformly be dispersed within the carbonized radioactive contaminant "B." Accordingly, the radioactivity level of the radioactive material on even a large amount of the carbonized radioactive contaminant "B" can uniformly and efficiently be reduced.

The radioactivity level of the carbonized radioactive contaminant "B" is not weak enough just after the carbonized radioactive contaminant "B" is stirred to uniformly disperse the photosynthetic bacteria. Thereafter, however, a discharge feeder 42 disposed below the stirring container 41 allows the carbonized radioactive contaminant "B" to safely be handled without direct contact and to be filled in the storage container 5. In a test measurement, the radioactivity level of the carbonized radioactive contaminant "B" thus filled in the storage container 5 was 3500 Bq/Kg upon the start of storage, but the subsequent action of the photosynthetic bacteria reduced the value to 81 Bq/Kg within two months. Accordingly, this test shows a significant effect of reducing the radioactivity level to under 100 Bq/Kg, a standard radioactive level of general food products within two months.

The photosynthetic bacteria was readily available at low cost as at least one of purple sulfur bacteria, purple nonsulfur bacteria and green sulfur bacteria. Accordingly, they are useful in reducing costs for decontaminating a radioactive material. The photosynthetic bacteria may include one or more types of purple sulfur bacteria, purple nonsulfur bacteria and green sulfur bacteria. Preferably, the photosynthetic bacteria is purple nonsulfur bacteria due to its high capability to reduce the radioactivity level.

Further, the carbonized radioactive contaminant "B" through a carbonization process in the thermal decomposition incinerator 1 is used in the present invention. This property enables the carbonized radioactive contaminant "B" to be decontaminated using the photosynthetic bacteria by increasing the density and intensity of the radioactivity of the carbonized radioactive contaminant. Accordingly, the radioactivity level can extremely efficiently be reduced.

The thermal decomposition incinerator 1 comprises a heating container 11 for stirring and heating the radioactive contaminant "A" in a sealed state. This property can prevent the release of exhaust gas from gas generated in the process of carbonization by a thermal decomposition into the atmosphere, as well as the radioactive material.

In a deodorizing apparatus 2, the radioactive material and odor contained in exhaust gas generated in the thermal decomposition incinerator 1 can be removed from the exhaust gas by introducing the exhaust gas and allowing the exhaust gas to pass through a first space 21 having sprayed water falling down and then a second space 22 filled with porous materials 22a. Accordingly, release of the radioactive material and odor into the atmosphere can be prevented, as well as the exhaust gas generated in the thermal decomposition incinerator 1. The radioactivity level of a radioactive material contained in the water stored in the water storage tank 21f after washing with sprayed water and a radioactive material on the porous materials 22a can readily be reduced using photosynthetic bacteria.

Further, in a smoke removing apparatus 3, the radioactive material and smoky microparticles can further be removed from exhaust gas discharged from the deodorizing apparatus 2 by introducing the exhaust gas discharged from the deodorizing apparatus 2 and allowing the exhaust gas to pass through water in a water tank 31 and then first and second smoke removing filter portions 32a, 32b of a filter chamber 32. Accordingly, the exhaust gas can be treated as completely harmless and released into the atmosphere.

Meanwhile, the above-configured radioactive decontamination method comprises the steps of: spraying an aqueous solution 44 containing photosynthetic bacteria over the carbonized radioactive contaminant "B"; stirring the carbonized radioactive contaminant "B" for a predetermined period of time; filling the storage container 5 with the stirred carbonized radioactive contaminant "B"; and storing the same for a predetermined period of time. Accordingly, the carbonized radioactive contaminant "B" can extremely readily be decontaminated.

The carbonized radioactive contaminant "B," with increased density and intensity of the radioactivity, is decontaminated by the photosynthetic bacteria, thereby extremely efficiently reducing the intensity of the radioactivity.

In the above embodiment, a carbonized radioactive contaminant "B" is chosen to be decontaminated by photosynthetic bacteria, but it may be a radioactive contaminant "A" prior to carbonization. This property can save the trouble of using the above-mentioned thermal decomposition incinerator 1, tar removing apparatus 15, deodorizing apparatus 2 and smoke removing apparatus 3.

Since the stirring container 41 contains an anaerobe such as purple sulfur bacteria, purple nonsulfur bacteria and green sulfur bacteria as photosynthetic bacteria, an oxygen-starved state is preferably retained in the stirring container 41. This oxygen-starved state can be achieved by preparing sucking means for sucking the air from the stirring container 41 (e.g., vacuum pump). This can also be achieved by preparing atmospheric gas supply means for supplying gas containing no oxygen (atmospheric gas) such as nitrogen, carbon dioxide and vapor to the stirring container 41.

Accordingly, the environment for growing purple sulfur bacteria, purple nonsulfur bacteria and green sulfur bacteria as an anaerobe can favorably be maintained by preparing an oxygen-starved state in the stirring container 41. This property improves the effect of reducing the radioactivity level.

Preferably, the carbonized radioactive contaminant "B" generated in the thermal decomposition incinerator 1 is fed by a feeder from the thermal decomposition incinerator 1 into the stirring container 41 without external exposure.

Further, the aqueous solution 44 may gravitationally be fed into a nozzle 45 by placing a tank 47 at a higher position than the nozzle 45. This property can save the trouble of using a pump 48a and a pressure control valve 48b. In this case, the flow control valve 48c may be a throttle valve having stop valve function.

### EXPLANATIONS OF LETTERS AND NUMERALS

- 1: Thermal decomposition incinerator
- 2: Deodorizing apparatus
- 3: Smoke removing apparatus
- 11: Heating container
- 21: First space
- 22: Second space
- 22a: porous material
- 31: Water tank
- 32a: First smoke removing filter portion (smoke removing filter)
- 32b: Second smoke removing filter portion (smoke removing filter)
- 41: Stirring container
- 42: Discharge feeder
- 44: Aqueous solution containing photosynthetic bacteria
- 45: Nozzle
- A: Radioactive contaminant
- B: Carbonized radioactive contaminant

## Claims

1. A radioactive decontamination device for reducing the radioactivity level of a radioactive contaminant having a radioactive material, comprising: a stirring container for stirring the radioactive contaminant; and a discharge feeder for discharging the stirred radioactive contaminant which is disposed below the stirring container, wherein the stirring container is provided with a nozzle for spraying an aqueous solution containing photosynthetic bacteria over the radioactive contaminant to be fed into the stirring container.

2. The radioactive decontamination device according to claim 1, wherein the photosynthetic bacteria is at least one of purple sulfur bacteria, purple nonsulfur bacteria and green sulfur bacteria.

3. The radioactive decontamination device according to claim 1 or 2, wherein the stirring container can be retained in an oxygen-starved state.

4. The radioactive decontamination device according to any one of claims 1 to 3, wherein the radioactive contaminant is a carbonized radioactive contaminant through a carbonization process in a thermal decomposition incinerator.

5. The radioactive decontamination device according to claim 4, wherein the thermal decomposition incinerator comprises a heating container for stirring and heating the radioactive contaminant in a sealed state.

6. The radioactive decontamination device according to claim 4 or 5, further comprising a deodorizing apparatus for removing the radioactive material and odor from exhaust gas generated in the thermal decomposition incinerator by introducing the exhaust gas and allowing the introduced exhaust gas to pass through a first space having sprayed water falling down and a second space having porous materials in sequence.

7. The radioactive decontamination device according to claim 6, further comprising a smoke removing apparatus for removing smoky microparticles from exhaust gas discharged from the deodorizing apparatus by introducing the exhaust gas and allowing the introduced exhaust gas to pass through stored water and then a smoke removing filter.

8. A radioactive decontamination method for reducing the radioactivity level of a radioactive contaminant having a radioactive material, comprising the steps of: spraying the radioactive contaminant with an aqueous solution containing photosynthetic bacteria; stirring the radioactive contaminant for a predetermined period of time; filling a container with the stirred radioactive contaminant; and storing the same for a predetermined period of time to reduce the radioactivity level of the radioactive contaminant.

9. The radioactive decontamination method according to claim 8, wherein the photosynthetic bacteria is at least one of purple sulfur bacteria, purple nonsulfur bacteria and green sulfur bacteria.

10. The radioactive decontamination method according to claim 8 or 9, wherein the radioactive contaminant is stirred and stored in an oxygen-starved state.

11. The radioactive decontamination method according to any one of claims 8 to 10, wherein the radioactive contaminant is a carbonized radioactive contaminant through a carbonization process.
